(19) Europäisches Patentamt
European Patent Office
Office européen des brevets

(11) **EP 4 759 293 A1**

(12) **EUROPEAN PATENT APPLICATION**
published in accordance with Art. 153(4) EPC

(43) Date of publication:
**17.06.2026 Bulletin 2026/25**

(21) Application number: **24851939.9**

(22) Date of filing: **08.08.2024**

(51) International Patent Classification (IPC):
***A61K 6/836*** (2020.01)   ***A61K 6/62*** (2020.01)
***A61K 6/887*** (2020.01)

(52) Cooperative Patent Classification (CPC):
**A61K 6/62; A61K 6/836; A61K 6/887**

(86) International application number:
**PCT/JP2024/028583**

(87) International publication number:
**WO 2025/033522 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.08.2023 JP 2023131556**

(71) Applicant: **Kuraray Noritake Dental Inc.**
**Kurashiki-shi, Okayama 710-0801 (JP)**

(72) Inventors:
- **ARAKI Mao**
  **Saitama-shi, Saitama 330-8565 (JP)**
- **SASAKI Ryoichi**
  **Kurashiki-shi, Okayama 713-8550 (JP)**
- **SHIMIZU Satoki**
  **Tainai-shi, Niigata 959-2653 (JP)**
- **KUDO Yasutaka**
  **Tainai-shi, Niigata 959-2653 (JP)**
- **TERAKAWA Eiichi**
  **Tainai-shi, Niigata 959-2653 (JP)**

(74) Representative: **D Young & Co LLP**
**3 Noble Street**
**London EC2V 7BQ (GB)**

(54) **CURABLE COMPOSITION FOR DENTAL USE**

(57)    The present invention provides a curable composition for dental use that possesses ion-releasing properties and aesthetic quality, and exhibits superior mechanical strength and tooth-strengthening ability. The present invention relates to a curable composition for dental use, comprising a glass (A), a polymerizable monomer (B), and a polymerization initiator (C), wherein the glass (A) comprises strontium, zinc, boron, and fluorine, and the strontium content is 8 to 33 mass%, the zinc content is 2.0 to 25 mass%, the boron content is 1.0 to 11 mass%, the fluorine content is 9 to 35 mass%, and the sodium content is 0 to 0.5 mass%, expressed as mass percentages of the constituent elements in the total mass of the glass (A).

**EP 4 759 293 A1**

**Description**

TECHNICAL FIELD

The present invention relates to curable compositions for dental use suited for dental restorative filling materials, dental cements, and dental core build-up materials.

BACKGROUND ART

Dental composite resins, composed primarily of polymerizable monomers, fillers, and polymerization initiators, have become the most widely used dental materials for the restoration of tooth defects and caries, as well as for the luting of dental prostheses, with dental restorative filling materials, dental cements, and dental core build-up materials representing examples of such materials. These dental composite resins have areas where improvement is desired, namely enhanced mechanical strength and reduced polymerization shrinkage during curing in the cured product.

Secondary caries occur due to bacterial penetration into the construction gaps formed by inadequate treatment, debonding of dental composite resin at the bonded surface, or degradation of dental composite resin at the bonded surface. It is strongly desired to minimize the occurrence of these secondary caries.

A common method for preventing secondary caries involves reinforcing the tooth structure through the formation of an acid-resistant layer. This reinforcement of tooth structure can be achieved by supplying ions, such as fluoride ions, to the tooth structure, specifically by incorporating ion-releasing compounds into dental composite resins, or by using materials prepared by mixing inorganic compounds known as glass ionomers with polycarboxylic acids.

However, incorporating ion-releasing compounds into dental composite resins not only results in inadequate ion release but also impairs strength and bond durability due to water absorption or other factors. Another issue is that glass ionomers exhibit low strength, though they exhibit superior ion-releasing properties.

As a technique for providing strength while preventing secondary caries, for example, Non Patent Literature 1 describes a dental composite resin that employs a urethane bond-containing dimethacrylate, 2-hydroxyethyl methacrylate, polycarboxylic acid, and fluoroaluminosilicate glass.

CITATION LIST

Non Patent Literature

Non Patent Literature 1: Osvaldo Zmener, et al., "Resistance against bacterial leakage of four luting agents used for cementation of complete cast crowns", American Journal of Dentistry, (US), February, 2014, Vol.27, No.1, p.51-55

SUMMARY OF INVENTION

Technical Problem

However, investigation by the present inventors revealed that the dental curable composition described in Non Patent Literature 1 involves low mechanical strength, though it can exhibit a certain level of ion-releasing properties. There are also areas for improvement in its ability to strengthen the tooth structure through ion release.

It is accordingly an object of the present invention to provide a curable composition for dental use that possesses ion-releasing properties and aesthetic quality, and exhibits superior mechanical strength and tooth-strengthening ability.

Solution to Problem

Specifically, the present invention includes the following.

[1] A curable composition for dental use, comprising a glass (A), a polymerizable monomer (B), and a polymerization initiator (C),

wherein the glass (A) comprises strontium, zinc, boron, and fluorine, and
the strontium content is 8 to 33 mass%, the zinc content is 2.0 to 25 mass%, the boron content is 1.0 to 11 mass%, the fluorine content is 9 to 35 mass%, and the sodium content is 0 to 0.5 mass%, expressed as mass percentages of the constituent elements in the total mass of the glass (A).

[2] The curable composition for dental use according to [1], wherein the polymerizable monomer (B) comprises a hydrophilic (meth)acrylic monomer (B-1) containing two or more (meth)acryloyloxy groups within a single molecule, and a hydrophobic (meth)acrylic monomer (B-2) containing two or more (meth)acryloyloxy groups within a single molecule.

[3] The curable composition for dental use according to [1] or [2], wherein the polymerizable monomer (B) further comprises a mono(meth)acrylic monomer (B-3) that does not contain any of a hydroxyl group, an acidic group, an amino group, or an amide group within a single molecule.

[4] The curable composition for dental use according to any one of [1] to [3], which comprises the following in total 100 parts by mass of the polymerizable monomer (B):

> 5 to 55 parts by mass of a hydrophilic (meth)acrylic monomer (B-1) containing two or more (meth)acryloyloxy groups within a single molecule;
> 25 to 70 parts by mass of a hydrophobic (meth)acrylic monomer (B-2) containing two or more (meth)acryloyloxy groups within a single molecule; and
> 0 to 55 parts by mass of a mono(meth)acrylic monomer (B-3) that does not contain any of a hydroxyl group, an acidic group, an amino group, or an amide group within a single molecule.

[5] The curable composition for dental use according to any one of [1] to [4],

> wherein the glass (A) further comprises aluminum and silicon, and
> the ratio of the amount of eluted strontium ions ($W_{Sr}$) to the sum of the amounts of eluted aluminum ions and eluted silicon ions ($W_{Al+Si}$) is 10 or more (($W_{Sr}$)/($W_{Al+Si}$)) when a silane-treated glass powder obtained by surface treatment of 100 parts by mass of the glass (A) in pulverized form with 1.5 parts by mass of $\gamma$-methacryloyloxypropyltrimethoxysilane is immersed in 37°C distilled water for 24 hours at a concentration of 5 mass%.

[6] The curable composition for dental use according to any one of [1] to [5], which is essentially free of polyacids.

[7] The curable composition for dental use according to any one of [1] to [6], wherein the polymerization initiator (C) comprises a chemical polymerization initiator (C-2).

[8] The curable composition for dental use according to any one of [1] to [7], which further comprises a polymerization accelerator (D).

[9] The curable composition for dental use according to any one of [1] to [8], which comprises a first agent and a second agent in separate packages.

[10] The curable composition for dental use according to [9], wherein the first agent comprises a chemical polymerization initiator (C-2), and the second agent comprises a polymerization accelerator (D).

Advantageous Effects of Invention

According to the present invention, a curable composition for dental use can be provided that possesses ion-releasing properties and aesthetic quality, and exhibits superior mechanical strength and tooth-strengthening ability.

The present invention can also provide a curable composition for dental use that also exhibits excellent radiopacity.

DESCRIPTION OF EMBODIMENTS

The following describes the present invention in detail through embodiments.

In this specification, the upper limits and lower limits of numeric ranges (for example, ranges of contents of components, ranges of values calculated from components, and ranges of physical properties) can be appropriately combined. In this specification, the numeric values of the symbols in the formulae can also be combined as appropriate.

Specifically, in this specification, the lower and upper limits of numeric ranges presented in a stepwise fashion may be combined independently. As an example, in a statement such as "preferably 10 to 90, more preferably 30 to 60" for the same parameter, it is possible to combine the preferred lower limit (10) and the more preferred upper limit (60) to form a range of "10 to 60."

Concerning numeric ranges, it is also possible to define only the lower limit without specifying the upper limit, for example, such as "10 or more" or "30 or more", based on a statement "preferably 10 to 90, more preferably 30 to 60." Similarly, it is possible to define only the upper limit without specifying the lower limit, such as "90 or less" or "60 or less."

Unless specifically indicated otherwise, when the expression of a numeric range is simply denoted as "10 to 90", it indicates 10 or more and 90 or less.

Similarly, for example, from statements presented for the same parameter such as "preferably 10 or more, more preferably 30 or more" and "preferably 90 or less, more preferably 60 or less", it is possible to combine the preferred lower limit (10) and the more preferred upper limit (60) to form the range of "10 or more and 60 or less." Furthermore, as with the foregoing, it is also possible to specify only the lower limit as "10 or more" or "30 or more", and, similarly, only the upper limit as "90 or less" or "60 or less."

As used herein, the term "(meth)acryl" is intended to be inclusive of both methacryl and acryl, and the same applies to similar expressions such as "(meth)acrylate", "(meth)acrylic acid ester", "(meth)acrylamide", and "(meth)acryloyloxy."

As used herein, the term "(meth)acrylic monomer" or "(meth)acrylic compound" is intended to be inclusive of both "(meth)acrylic acid ester compounds" and "(meth)acrylamides and their derivatives."

As used herein, the term "polymerizable monomer" is used to refer to a (meth)acrylic compound polymerized by the polymerization initiator (C) described below.

As used herein, unless specifically indicated otherwise, "curability" and "storage stability" refer to the "curability" and "storage stability" concerning the curable composition for dental use according to an aspect of the present invention.

As used herein, unless specifically indicated otherwise, "ion-releasing properties" refers to the "silicon ion-releasing properties", "aluminum ion-releasing properties", "zinc ion-releasing properties", and "strontium ion-releasing properties" concerning a cured product of the curable composition for dental use according to an aspect of the present invention.

As used herein, unless specifically indicated otherwise, "mechanical strength" or "strength" refers to the "flexural strength" and "flexural modulus" of a cured product of the curable composition for dental use according to an aspect of the present invention.

As used herein, unless specifically indicated otherwise, "tooth-strengthening ability" refers to the "tooth-strengthening ability" of a cured product of the curable composition for dental use according to an aspect of the present invention. The tooth-strengthening ability serves as an index of resistance to acid-induced demineralization, and is calculated in terms of "demineralization inhibition rate."

[Curable Composition for Dental Use]

A curable composition for dental use of the present invention comprises a glass (A), a polymerizable monomer (B), and a polymerization initiator (C).

Preferably, the polymerizable monomer comprises a hydrophilic (meth)acrylic monomer (B-1) containing two or more (meth)acryloyloxy groups within a single molecule, and a hydrophobic (meth)acrylic monomer (B-2) containing two or more (meth)acryloyloxy groups within a single molecule. More preferably, the polymerizable monomer comprises a mono(meth)acrylic monomer (B-3) that does not contain any of a hydroxyl group, an acidic group, an amino group, or an amide group within a single molecule.

The following describes each component of a curable composition for dental use of the present invention.

<Glass (A)>

In the curable composition for dental use, the glass (A) is used to impart strength and ion-releasing properties to the cured product of the curable composition for dental use.

The glass (A) comprises strontium, zinc, boron, and fluorine.

The presence of strontium, zinc, and fluorine allows for the release of strontium ions, zinc ions, and fluoride ions. By being incorporated into the tooth structure, these ions can improve the tooth's resistance to acid-induced demineralization.

The glass (A) comprises silicon and aluminum as its basic structure, with boron believed to control the strength of this basic structure. It is speculated that an overly rigid basic structure limits ion release and reduces tooth-strengthening ability, whereas an excessively fragile basic structure results in decreased strength.

The content of each component in the glass (A) is as follows: 8 to 33 mass% strontium, 2.0 to 25 mass% zinc, 1.0 to 11 mass% boron, 9 to 35 mass% fluorine, and 0 to 0.5 mass% sodium, expressed as mass percentages of the constituent elements in the total mass of the glass (A).

In the following, the content of each component refers to the mass percent of each constituent element in the total mass of the glass (A).

Although the mechanism by which strontium enhances tooth-strengthening ability remains unclear, the following speculation has been made.

It is presumed that strontium ions partially substitute for calcium ions present in the tooth structure, allowing it to exhibit acid resistance, thereby reducing the amount of calcium ions that leach out of the tooth structure due to demineralization.

When the strontium content is less than 8 mass%, it may lead to a decrease in tooth-strengthening ability. When the content exceeds 33 mass%, crystals become more likely to precipitate during glass production, potentially causing the glass to devitrify.

In view of superior tooth-strengthening ability, the strontium content is preferably 9 to 32 mass%, more preferably 10 to 31 mass%, even more preferably 10.5 to 30 mass%, particularly preferably 11 to 29 mass%.

The mechanism by which zinc enhances tooth-strengthening ability also remains elusive. However, it is speculated that zinc ions also partially substitute for calcium ions present in the tooth structure, imparting acid resistance to the tooth structure and reducing the amount of calcium ions that leach out of the tooth structure due to demineralization, thereby acting to enhance tooth-strengthening ability.

When the zinc content is less than 2.0 mass%, it may result in a decrease in tooth-strengthening ability. When the content exceeds 25 mass%, crystal precipitation becomes more likely to occur during glass production, potentially leading to glass devitrification.

In view of superior tooth-strengthening ability, the zinc content is preferably 2.5 to 23 mass%, more preferably 3 to 20 mass%, even more preferably 3.5 to 18 mass%, particularly preferably 3.5 to 16 mass%.

In view of achieving both strength and sustained ion-releasing properties, and controlling the strength of the basic structure to regulate tooth-strengthening ability, as well as preventing devitrification during glass production, the boron content is 1.0 to 11 mass%, preferably 1.2 to 10 mass%, more preferably 1.5 to 9.5 mass%, even more preferably 1.7 to 9.3 mass%, particularly preferably 2.0 to 9.0 mass%.

The mechanism by which fluorine enhances tooth-strengthening ability appears to involve the reaction of released fluoride ions with the hydroxyapatite ($Ca_{10}(PO_4)_6(OH_2)$) in the tooth structure, converting it into fluoroapatite ($Ca_{10}(PO_4)_6(F_2)$) and thereby improving the acid resistance of the tooth structure.

When the fluorine content is less than 9 mass%, the refractive index of glass (A) may exceed 1.6, which may complicate adjustment for the preparation of the curable composition for dental use. When the fluorine content exceeds 35 mass%, crystals become more likely to precipitate during glass production, potentially causing the glass to devitrify.

In view of superior tooth-strengthening ability, the fluorine content is preferably 10 to 34 mass%, more preferably 10.5 to 33 mass%, even more preferably 11 to 32 mass%, particularly preferably 12 to 30 mass%.

When the sodium content in glass (A) exceeds 0.5 mass%, crystal precipitation becomes more likely to occur during glass production, potentially leading to glass devitrification. From this viewpoint, the sodium content is 0 to 0.5 mass%.

The sodium content is preferably 0 to 0.3 mass%, more preferably 0 to 0.1 mass%, even more preferably 0 to 0.01 mass%, particularly preferably 0 to 0.001 mass%.

In a certain preferred embodiment, the sodium content may be 0 mass%.

Any known alkali metals (lithium, potassium, rubidium, cesium, francium) may be used without restriction as alkali metals other than sodium.

Such alkali metals other than sodium may have the same content as sodium.

When the content of alkali metals exceeds 0.5 mass%, crystals become more likely to precipitate during glass production, potentially causing the glass to devitrify. From this viewpoint, the total content of alkali metals is preferably 0 to 0.5 mass%.

Preferably, glass (A) comprises aluminum and silicon.

The aluminum content is not particularly limited, as long as the present invention can exhibit its effects. However, in view of achieving both strength and sustained ion-releasing properties and preventing devitrification during glass production, the aluminum content is preferably 2 to 15 mass%, more preferably 2.1 to 14 mass%, even more preferably 2.2 to 13 mass%, particularly preferably 2.3 to 12.5 mass%.

The silicon content is not particularly limited, as long as the present invention can exhibit its effects. However, in view of achieving both strength and sustained ion-releasing properties and preventing devitrification during glass production, the silicon content is preferably 5 to 25 mass%, more preferably 6 to 24 mass%, even more preferably 6.5 to 22 mass%, particularly preferably 7 to 20 mass%.

A certain preferred embodiment is, for example, a curable composition for dental use in which the glass (A) comprises 5 to 25 mass% of silicon, 2 to 15 mass% of aluminum, and 1.0 to 11 mass% of boron.

In a curable composition for dental use of the present invention, the ratio of the amount of eluted strontium ions ($W_{Sr}$) to the sum of the amounts of eluted aluminum ions and eluted silicon ions ($W_{Al+Si}$) is preferably 10 or more ($(W_{Sr})/(W_{Al+Si})$) when a silane-treated glass powder obtained by surface treatment of 100 parts by mass of glass (A) in pulverized form with 1.5 parts by mass of γ-methacryloyloxypropyltrimethoxysilane is immersed in 37°C distilled water for 24 hours at a concentration of 5 mass%.

The ratio of eluted ion amounts ($W_{Sr}$)/($W_{Al+Si}$) is more preferably 12 or more, even more preferably 15 or more, particularly preferably 18 or more, most preferably 25 or more.

The method for measuring the ratio of eluted ion amounts ($W_{Sr}$)/($W_{Al+Si}$) is as described in the EXAMPLES below.

In view of properties such as radiopacity and sustained ion-release, the glass (A) may comprise other components.

Examples of such additional components include titanium, barium, ytterbium, hafnium, zirconium, yttrium, tin, tantalum, antimony, vanadium, cerium, europium, praseodymium, tungsten, germanium, niobium, lanthanum, copper, iron, bismuth, chromium, manganese, cobalt, terbium, phosphorus, and alkali earth metals (calcium, barium, radium, beryllium, magnesium).

The content of additional components is preferably less than 3 mass%, more preferably less than 1 mass%, even more preferably less than 0.1 mass%, particularly preferably less than 0.001 mass%.

The balance in glass (A) is oxygen.

Any known technique can be employed without restriction to quantify the components contained in glass (A). For example, X-ray fluorescence may be used for quantification of the components contained in glass (A).

The shape of glass (A) is not particularly limited, and may take any particle form, including spherical, needle-shaped, plate-like, crushed, or scale-like. The glass (A) may be dissolved in a polymerizable monomer or the like. In view of considerations such as the handling properties of the curable composition for dental use, and the ion-releasing properties, mechanical strength, and transparency of its cured product, it is preferable that the glass (A), when it is particulate, have an average primary particle diameter of preferably 0.001 to 10 $\mu$m, more preferably 0.005 to 8.0 $\mu$m, even more preferably 0.01 to 6.0 $\mu$m, particularly preferably 1.0 to 5.0 $\mu$m. Any known method can be employed to obtain a glass (A) of a predetermined particle diameter. For example, it is possible to employ methods such as pulverization using a ball mill or a bead mill.

In this specification, the average primary particle diameter of glass (A) can be determined using techniques such as a laser diffraction scattering method, light microscopy, and electron microscopy. More specifically, a laser diffraction scattering method and light microscopy are more convenient for particles with a particle size of 100 nm or larger, whereas electron microscopy is a more convenient method of particle diameter measurement for particles smaller than 100 nm.

In view of aesthetic quality as a curable composition for dental use, the glass (A) has a refractive index of preferably 1.500 to 1.600.

When the refractive index is below 1.500 or above 1.600, the difference in refractive index with respect to polymerizable monomer (B) becomes too large, potentially lowering the translucency of the curable composition for dental use and compromising its aesthetic quality. The refractive index of the glass is measured following the refractive index measurement method of JIS B 7071-2:2018.

To adjust compatibility with polymerizable monomer (B), the glass (A) may be used after surface treatment with a known surface treatment agent such as polysiloxanes, polyethylene glycol, fatty acid amides, or silane coupling agents, within a range that does not compromise the ion-releasing properties. However, it is preferable to avoid silica coating or other strong coating processes that cover ion-releasing compounds, or the formation of core-shell structures because these processes impair the ion-releasing properties.

Any known method may be employed for the surface treatment without restriction. Examples include (1) forming a layer of silanol compound on the surface of glass (A), followed by intermolecular dehydrocondensation of the silanol groups; and (2) (partially) hydrolyzing a hydrolyzable silane compound to form an oligomer through intermolecular dehydrocondensation of the silanol groups, adding a powdery ion-releasing compound to the siloxane oligomer to form a siloxane oligomer layer on the surface of the ion-releasing compound, and polymerizing the siloxane oligomer through intermolecular dehydrocondensation.

In method (1), a hydrolyzable silane compound and the required amount of water are added into a water-miscible organic solvent such as methanol, ethanol, or t-butanol, and the hydrolyzable silane compound is (partially) hydrolyzed in the presence of an acid catalyst to prepare an organic solution containing the silanol compound produced by hydrolysis.

Examples of the silane compound include:

vinyl group-containing silane compounds such as vinyltrimethoxysilane, vinyltriethoxysilane, vinyltrichlorosilane, and vinyltri($\beta$-methoxyethoxy)silane;

(meth)acryloyl group-containing silane compounds such as $\gamma$-(meth)acryloyloxypropyltrimethoxysilane, 8-(meth)acryloyloxyoctyltrimethoxysilane, 11-(meth)acryloyloxyundecyltrimethoxysilane, $\gamma$-(meth)acryloyloxypropylmethyl-dimethoxysilane, $\gamma$-(meth)acryloyloxypropylmethyldiethoxysilane, and $\gamma$-(meth)acryloyloxypropyldimethylmono-methoxysilane;

amino group-containing silane compounds such as $\gamma$-aminopropyltriethoxysilane;

epoxy group-containing silane compounds such as $\gamma$-glycidoxypropyltrimethoxysilane; and

mercapto group-containing silane compounds such as $\gamma$-mercaptopropyltrimethoxysilane.

The organic solution containing silanol compounds may alternatively be prepared by (partially) hydrolyzing a hydrolyzable silane compound with an excess of added water in the presence of an acid catalyst to form an aqueous solution containing the silanol compound, followed by extraction of the silanol compound in the aqueous solution using a water-immiscible organic solvent such as ethyl acetate, ethyl ether, chloroform, or methylene chloride as the extracting solvent. Subsequently, glass (A) is added in powder form into the organic solution obtained by using the above methods, and the organic solvent is evaporated by heating or under reduced pressure to yield a glass (A) comprising composite particles with the silanol compound adhered on the glass surface in a layered fashion. After optionally adding an acid or base, the glass (A) is heated to induce intermolecular dehydrocondensation of the silanol groups, forming a glass (A) comprising composite particles with a polysiloxane layer formed on the glass surface.

In method (2), a hydrolyzable silane compound is (partially) hydrolyzed with a predetermined amount of added water in the presence of an acid catalyst while removing the by-product alcohol by distillation, yielding a siloxane oligomer. To this siloxane oligomer, glass (A) is added in powder form to form a siloxane oligomer layer on the surface of glass particles. After optionally adding an acid or base, the resulting material is heated to induce intermolecular dehydrocondensation of the silanol groups in the siloxane oligomer, forming a glass (A) comprising composite particles with a polysiloxane layer formed on the surface of substrate glass particles.

The content of the glass (A) in the curable composition for dental use is not particularly limited, as long as the present invention can exhibit its effects. However, in view of allowing the present invention to more readily exhibit its effects, the content of glass (A) is preferably 30 parts or more by mass, more preferably 40 parts or more by mass, even more preferably 50 parts or more by mass, and is preferably 1,000 parts or less by mass, more preferably 900 parts or less by mass, even more preferably 850 parts or less by mass relative to total 100 parts by mass of the polymerizable monomer (B).

In certain preferred embodiments, the content of the glass (A) in the curable composition for dental use is preferably 20 to 95 mass%, more preferably 25 to 94 mass% in total 100 mass% of the curable composition for dental use. In view of achieving even superior ion-releasing properties in the cured product, the content of glass (A) is even more preferably 30 to 92 mass%, particularly preferably 35 to 90 mass%.

<Hydrophilic (Meth)Acrylic Monomer (B-1) Containing Two or More (Meth)Acryloyloxy Groups within Single Molecule>

As the polymerizable monomer (B) component, a curable composition for dental use of the present invention preferably comprises a hydrophilic (meth)acrylic monomer (B-1) containing two or more (meth)acryloyloxy groups within a single molecule (hereinafter, also referred to simply as "hydrophilic polyfunctional (meth)acrylic monomer (B-1)").

The hydrophilic polyfunctional (meth)acrylic monomer (B-1) refers to a polyfunctional polymerizable monomer with a solubility of 5 mass% or more in water at 25°C, preferably one with a solubility of 10 mass% or more, more preferably one with a solubility of 15 mass% or more in water at 25°C.

In the curable composition for dental use, the hydrophilic polyfunctional (meth)acrylic monomer (B-1) is used to impart adhesive properties to the curable composition for dental use, and ion-releasing properties to the cured product of the curable composition for dental use.

The hydrophilic polyfunctional (meth)acrylic monomer (B-1) increases ion conductivity, promoting the incorporation of released ions into the tooth structure, thereby enhancing the tooth-strengthening ability through integrated action with components such as strontium ions and zinc ions.

By containing two or more (meth)acryloyloxy groups within a single molecule, the hydrophilic polyfunctional (meth)acrylic monomer (B-1) can also provide superior mechanical strength in the cured product.

The number of (meth)acryloyloxy groups within a single molecule of hydrophilic polyfunctional (meth)acrylic monomer (B-1) is not particularly limited, as long as two or more (meth)acryloyloxy groups are present.

The hydrophilic polyfunctional (meth)acrylic monomer (B-1) has hydrophilic groups such as hydroxyl groups, oxymethylene groups, oxyethylene groups, oxypropylene groups, and amide groups.

Examples of the hydrophilic polyfunctional (meth)acrylic monomer (B-1) include polyfunctional (meth)acrylate compounds such as glycerol di(meth)acrylate, 1,2-bis(3-(meth)acryloyloxy-2-hydroxypropoxy)ethane, 2,2-bis[4-(3-(meth)acryloyloxy)-2-hydroxypropoxyphenyl]propane, and pentaerythritol tri(meth)acrylate. These may be used alone, or two or more thereof may be used in combination.

In view of improving the bond durability of the curable composition for dental use to the tooth structure and enhancing the ion-releasing properties of the cured product, preferred are bifunctional (meth)acrylate compounds such as glycerol di(meth)acrylate, and 1,2-bis(3-(meth)acryloyloxy-2-hydroxypropoxy)ethane, more preferably glycerol dimethacrylate, and 1,2-bis(3-methacryloyloxy-2-hydroxypropoxy)ethane.

The content of the hydrophilic polyfunctional (meth)acrylic monomer (B-1) in the curable composition for dental use is preferably 1 to 60 parts by mass, more preferably 2 to 50 parts by mass in total 100 parts by mass of the polymerizable monomer (B) in the curable composition for dental use. In view of achieving even superior ion-releasing properties in the cured product, the content of hydrophilic polyfunctional (meth)acrylic monomer (B-1) is even more preferably 5 to 55 parts by mass, particularly preferably 8 to 40 parts by mass.

In a certain preferred embodiment, the content of the hydrophilic polyfunctional (meth)acrylic monomer (B-1) in the curable composition for dental use is preferably 1 to 60 mass%, more preferably 2 to 50 mass% in total 100 mass% of the curable composition for dental use. In view of achieving even superior ion-releasing properties in the cured product, the content of hydrophilic polyfunctional (meth)acrylic monomer (B-1) is even more preferably 2.5 to 55 mass%, particularly preferably 3 to 40 mass%.

<Hydrophobic (Meth)Acrylic Monomer (B-2) Containing Two or More (Meth)Acryloyloxy Groups within Single Molecule>

As the polymerizable monomer (B) component, a curable composition for dental use of the present invention preferably comprises a hydrophobic (meth)acrylic monomer (B-2) containing two or more (meth)acryloyloxy groups within a single molecule (hereinafter, also referred to simply as "hydrophobic polyfunctional (meth)acrylic monomer (B-2)").

The hydrophobic polyfunctional (meth)acrylic monomer (B-2) refers to a polyfunctional polymerizable monomer with a solubility of less than 5 mass% in water at 25°C.

In the curable composition for dental use, the hydrophobic polyfunctional (meth)acrylic monomer (B-2) is used to impart strength to the curable composition for dental use.

Examples of the hydrophobic polyfunctional (meth)acrylic monomer (B-2) include aromatic bifunctional polymerizable monomers, aliphatic bifunctional polymerizable monomers, and tri- and higher-functional polymerizable monomers.

Examples of the aromatic bifunctional polymerizable monomers include 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane (with an average of 2.6 moles of ethoxy group added, commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred among these are 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-(meth)acryloyloxypolyethoxyphenyl)propane (with an average of 2.6 moles of ethoxy group added, commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional polymerizable monomers include ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl)di(meth)acrylate. Preferred among these are triethylene glycol di(meth)acrylate, neopentyl glycol di(meth)acrylate, 1,2-bis(3-methacryloyloxy-2-hydroxypropoxy)ethane, and 2,2,4-trimethylhexamethylenebis(2-carbamoyloxyethyl)dimethacrylate (commonly known as UDMA).

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred among these is N,N-(2,2,4-trimethylhexamethylene)bis[2-(aminocarboxy)propane-1,3-diol]tetramethacrylate.

In view of mechanical strength and handling properties, preferred for use as hydrophobic polyfunctional (meth)acrylic monomers (B-2) are aromatic bifunctional polymerizable monomers and aliphatic bifunctional polymerizable monomers.

Preferred as the aromatic bifunctional polymerizable monomers are Bis-GMA and D-2.6E.

Preferred as the aliphatic bifunctional polymerizable monomers are triethylene glycol di(meth)acrylate, neopentyl glycol di(meth)acrylate, 1,6-hexanediol di(meth)acrylate, 1,10-decanediol di(meth)acrylate, 1,2-bis(3-methacryloyloxy-2-hydroxypropoxy)ethane, and UDMA.

The hydrophobic polyfunctional (meth)acrylic monomer (B-2) may be incorporated alone, or two or more thereof may be used in combination.

The content of hydrophobic polyfunctional (meth)acrylic monomer (B-2) is preferably 10 to 80 parts by mass, more preferably 20 to 75 parts by mass in total 100 parts by mass of the polymerizable monomer (B) in the curable composition for dental use. In view of achieving even superior mechanical strength in the cured product, the content of hydrophobic polyfunctional (meth)acrylic monomer (B-2) is even more preferably 25 to 70 parts by mass, particularly preferably 30 to 60 parts by mass.

When the content of hydrophobic polyfunctional (meth)acrylic monomer (B-2) is excessively high, it may lead to a decrease in ion conductivity and tooth-strengthening ability. When the content of hydrophobic polyfunctional (meth)acrylic monomer (B-2) is too low, it may not be possible to adequately produce the effect that improves the mechanical strength.

In a certain preferred embodiment, the content of the hydrophobic polyfunctional (meth)acrylic monomer (B-2) in the curable composition for dental use is preferably 1 to 60 mass%, more preferably 2 to 50 mass% in total 100 mass% of the curable composition for dental use. In view of providing even superior ion-releasing properties in the cured product, the content of hydrophobic polyfunctional (meth)acrylic monomer (B-2) is even more preferably 3 to 55 mass%, particularly preferably 4 to 40 mass%.

<Mono(Meth)Acrylic Monomer (B-3) That Does Not Contain Any of Hydroxyl Group, Acidic Group, Amino Group, or Amide Group within Single Molecule>

Preferably, the polymerizable monomer (B) in the curable composition for dental use further comprises a mono(meth)acrylic monomer (B-3) that does not contain any of a hydroxyl group, an acidic group, an amino group, or an amide group within a single molecule (hereinafter, also referred to simply as "mono(meth)acrylic monomer (B-3)").
In the curable composition for dental use, the mono(meth)acrylic monomer (B-3) can be suitably used in view of reducing the viscosity of the curable composition for dental use and improving its handling properties, and imparting an effect that improves the strength of the cured product.
Examples of the acidic group include phosphoric acid groups, sulfo groups, and carboxy groups.
Examples of the mono(meth)acrylic monomer (B-3) include aromatic monofunctional (meth)acrylic monomers, and aliphatic monofunctional (meth)acrylic monomers.
Examples of the aromatic monofunctional (meth)acrylic monomers include:

(meth)acrylates having two aromatic rings, such as o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, butoxylated o-phenylphenol (meth)acrylate, butoxylated m-phenylphenol (meth)acrylate, butoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 5-(o-phenoxyphenyl)pentyl (meth)acrylate, 5-(m-phenoxyphenyl)pentyl (meth)acrylate, 5-(p-phenoxyphenyl)pentyl (meth)acrylate, 6-(o-phenoxyphenyl)hexyl (meth)acrylate, 6-(m-phenoxyphenyl)hexyl (meth)acrylate, and 6-(p-phenoxyphenyl)hexyl (meth)acrylate; and
(meth)acrylates having one aromatic ring, such as benzyl (meth)acrylate, and phenyl (meth)acrylate.

Examples of the aliphatic monofunctional (meth)acrylic monomers include methyl (meth)acrylate, ethyl (meth)acrylate, propyl (meth)acrylate, isopropyl (meth)acrylate, n-butyl (meth)acrylate, sec-butyl (meth)acrylate, t-butyl (meth)acrylate, isobutyl (meth)acrylate, n-hexyl (meth)acrylate, cyclohexyl (meth)acrylate, 2-ethylhexyl (meth)acrylate, lauryl (meth)acrylate, cetyl (meth)acrylate, stearyl (meth)acrylate, isobornyl (meth)acrylate, 2,3-dibromopropyl (meth)acrylate, 3-(meth)acryloyloxypropyltrimethoxysilane, and 11-(meth)acryloyloxyundecyltrimethoxysilane. These may be used alone, or two or more thereof may be used in combination.
In view of achieving superior handling properties in the curable composition for dental use and superior strength in the cured product, preferred are ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, and lauryl (meth)acrylate, more preferably ethoxylated o-phenylphenol (meth)acrylate, ethoxylated m-phenylphenol (meth)acrylate, ethoxylated p-phenylphenol (meth)acrylate, o-phenoxybenzyl (meth)acrylate, m-phenoxybenzyl (meth)acrylate, p-phenoxybenzyl (meth)acrylate, and lauryl (meth)acrylate, even more preferably ethoxylated o-phenylphenol (meth)acrylate, ethoxylated m-phenylphenol (meth)acrylate, o-phenoxybenzyl (meth)acrylate, and m-phenoxybenzyl (meth)acrylate.
In these preferred embodiments, it is particularly preferable that the aforementioned compounds are in methacrylate form, most preferably ethoxylated o-phenylphenol methacrylate, ethoxylated m-phenylphenol methacrylate, o-phenoxybenzyl methacrylate, and m-phenoxybenzyl methacrylate.
The content of mono(meth)acrylic monomer (B-3) is preferably 0 to 60 parts by mass, more preferably 0 to 55 parts by mass in total 100 parts by mass of the polymerizable monomer (B) in the curable composition for dental use. In view of allowing the present invention to more readily exhibit its effects, the content of mono(meth)acrylic monomer (B-3) is even more preferably 0 to 50 parts by mass, particularly preferably 0 to 45 parts by mass.
In a certain preferred embodiment, when the curable composition for dental use comprises the mono(meth)acrylic monomer (B-3), the content of the mono(meth)acrylic monomer (B-3) is preferably 5 to 60 parts by mass, more preferably 8 to 55 parts by mass in total 100 parts by mass of the polymerizable monomer (B) in the curable composition for dental use. In view of allowing the present invention to more readily exhibit its effects, the content of mono(meth)acrylic monomer (B-3) is even more preferably 8 to 50 parts by mass, particularly preferably 10 to 45 parts by mass.

In another certain preferred embodiment, the content of the mono(meth)acrylic monomer (B-3) in the curable composition for dental use is preferably 0 to 60 mass%, more preferably 0 to 50 mass% in total 100 mass% of the curable composition for dental use. In view of providing even superior ion-releasing properties in the cured product, the content of mono(meth)acrylic monomer (B-3) is even more preferably 0 to 55 mass%, particularly preferably 0 to 40 mass%.

A certain preferred embodiment is, for example, a curable composition for dental use that comprises the following in total 100 parts by mass of the polymerizable monomer (B):

5 to 55 parts by mass of a hydrophilic (meth)acrylic monomer (B-1) containing two or more (meth)acryloyloxy groups within a single molecule;

25 to 70 parts by mass of a hydrophobic (meth)acrylic monomer (B-2) containing two or more (meth)acryloyloxy groups within a single molecule; and

0 to 55 parts by mass of a mono(meth)acrylic monomer (B-3) that does not contain any of a hydroxyl group, an acidic group, an amino group, or an amide group within a single molecule.

<Polymerization Initiator (C)>

The polymerization initiator (C) may be selected from polymerization initiators used in industry, preferably from those used in dentistry.

Preferably, the polymerization initiator (C) comprises at least one selected from the group consisting of a photopolymerization initiator (C-1) and a chemical polymerization initiator (C-2). More preferably, the polymerization initiator (C) comprises at least a photopolymerization initiator (C-1). The polymerization initiator (C) may comprise both a photopolymerization initiator (C-1) and a chemical polymerization initiator (C-2).

A certain preferred embodiment is, for example, a curable composition for dental use in which the polymerization initiator (C) comprises a chemical polymerization initiator (C-2).

Examples of the photopolymerization initiator (C-1) include (bis)acylphosphine oxides (including salts), thioxanthones (including salts such as quaternary ammonium salts), ketals, $\alpha$-diketones, coumarins, anthraquinones, benzoin alkyl ether compounds, and $\alpha$-aminoketone compounds. These may be used alone, or two or more thereof may be used in combination.

Preferred for use among these photopolymerization initiators (C-1) is at least one selected from the group consisting of (bis)acylphosphine oxides and $\alpha$-diketones. In this way, a curable composition can be obtained that has excellent photocurability both in the visible and near-ultraviolet regions, and that shows sufficient photocurability regardless of whether the light source used is a halogen lamp, a light emitting diode (LED), or a xenon lamp.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, sodium salts of 2,4,6-trimethylbenzoylphenylphosphine oxide, potassium salts of 2,4,6-trimethylbenzoylphenylphosphine oxide, and ammonium salts of 2,4,6-trimethylbenzoylphenylphosphine oxide.

Examples of bisacylphosphine oxides in the (bis)acylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,3,6-trimethylbenzoyl)-2,4,4-trimethylpentylphosphine oxide.

Other examples of the (bis)acylphosphine oxides include the compounds mentioned in JP2000-159621 A.

Among these (bis)acylphosphine oxides, preferred for use as photopolymerization initiator (C-1) are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the $\alpha$-diketones include diacetyl, benzyl, camphorquinone, 2,3-pentadione, 2,3-octadione, 9,10-phenanthrenequinone, 4,4'-oxybenzyl, and acenaphthenequinone. Particularly preferred is camphorquinone for its maximum absorption wavelength occurring in the visible light region.

Preferred for use as chemical polymerization initiator (C-2) are organic peroxides.

The organic peroxides used as chemical polymerization initiator (C-2) are not particularly limited, and may be known organic peroxides. Typical examples of organic peroxides include ketone peroxides, hydroperoxides, diacyl peroxides, dialkyl peroxides, peroxyketals, peroxyesters, and peroxydicarbonates. These may be used alone, or two or more thereof may be used in combination.

Examples of the ketone peroxides include methyl ethyl ketone peroxide, methyl isobutyl ketone peroxide, methyl cyclohexanone peroxide, and cyclohexanone peroxide.

Examples of the hydroperoxides include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butyl cumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and 4,4-bis(t-butylperoxy)valeric acid n-butyl ester.

Examples of the peroxyesters include $\alpha$-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxymaleate.

Examples of the peroxydicarbonates include di(3-methoxybutyl)peroxydicarbonate, di(2-ethylhexyl)peroxydicarbonate, bis(4-t-butylcyclohexyl)peroxydicarbonate, diisopropylperoxydicarbonate, di-n-propylperoxydicarbonate, di(2-ethoxyethyl)peroxydicarbonate, and diallyl peroxydicarbonate.

From an overall balance of safety, curability, and storage stability, more preferred for use as chemical polymerization initiator (C-2) among these organic peroxides is one selected from hydroperoxides and diacyl peroxides, even more preferably hydroperoxides. Among such organic peroxides, preferred for use as chemical polymerization initiator (C-2) are one or more selected from the group consisting of benzoyl peroxide, cumene hydroperoxide, t-butyl hydroperoxide, and 1,1,3,3-tetramethylbutyl hydroperoxide, more preferably one or more selected from the group consisting of cumene hydroperoxide, t-butyl hydroperoxide, and 1,1,3,3-tetramethylbutyl hydroperoxide, even more preferably 1,1,3,3-tetramethylbutyl hydroperoxide.

The content of polymerization initiator (C) is not particularly limited. However, in view of considerations such as the curability of the curable composition for dental use, the polymerization initiator (C) is preferably 0.001 to 30 parts by mass relative to total 100 parts by mass of polymerizable monomer (B). When the content of polymerization initiator (C) is 0.001 parts or more by mass, polymerization can sufficiently proceed, preventing stickiness.

In view of these, the content of polymerization initiator (C) is more preferably 0.03 parts or more by mass, even more preferably 0.05 parts or more by mass, particularly preferably 0.1 parts or more by mass relative to total 100 parts by mass of polymerizable monomer (B). When the content of polymerization initiator (C) is 30 parts or less by mass, it is possible to prevent precipitation or separation of polymerization initiator (C) from the curable composition for dental use. In view of these, the content of polymerization initiator (C) is more preferably 10 parts or less by mass, even more preferably 5 parts or less by mass, particularly preferably 2 parts or less by mass relative to total 100 parts by mass of polymerizable monomer (B).

When the polymerization initiator (C) comprises a photopolymerization initiator (C-1), the content of photopolymerization initiator (C-1) is not particularly limited. However, in view of allowing the present invention to more readily exhibit its effects, it is preferable in an embodiment of the curable composition for dental use that the content of the photopolymerization initiator (C-1) in polymerization initiator (C) is preferably 1 to 40 mass%, more preferably 5 to 30 mass%, even more preferably 8 to 20 mass% in total 100 mass% of the polymerization initiator (C).

When the polymerization initiator (C) comprises a chemical polymerization initiator (C-2), the content of chemical polymerization initiator (C-2) is not particularly limited. However, in view of allowing the present invention to more readily exhibit its effects, it is preferable in an embodiment of the curable composition for dental use that the content of the chemical polymerization initiator (C-2) in polymerization initiator (C) is preferably 60 to 100 mass%, more preferably 70 to 100 mass%, even more preferably 80 to 100 mass% in total 100 mass% of the polymerization initiator (C).

<Polymerization Accelerator (D)>

Preferably, the curable composition for dental use further comprises a polymerization accelerator (D).

Examples of the polymerization accelerator (D) include amines, sulfinic acid and salts thereof, sulfites, bisulfites, aldehydes, thiourea compounds, organic phosphorus compounds, borate compounds, barbituric acid and its derivatives, triazine compounds, vanadium compounds, copper compounds, tin compounds, cobalt compounds, halogen compounds, and thiol compounds. These may be used alone, or two or more thereof may be used in combination.

A certain preferred embodiment is, for example, a curable composition for dental use in which the polymerization accelerator (D) is one or more selected from the group consisting of amines, sulfinic acid and salts thereof, sulfites, bisulfites, aldehydes, thiourea compounds, organic phosphorus compounds, barbituric acid and its derivatives, triazine compounds, vanadium compounds, copper compounds, tin compounds, cobalt compounds, halogen compounds, and thiol compounds.

Another preferred embodiment is, for example, a curable composition for dental use that comprises a glass (A), a polymerizable monomer (B), a polymerization initiator (C), and a polymerization accelerator (D), and that is essentially free of polyacids,

> wherein the glass (A) comprises strontium, zinc, boron, and fluorine, and
> the strontium content is 8 to 33 mass%, the zinc content is 2.0 to 25 mass%, the boron content is 1.0 to 11 mass%, the fluorine content is 9 to 35 mass%, and the sodium content is 0 to 0.5 mass%, expressed as mass percentages of the constituent elements in the total mass of the glass (A), and
> the polymerization initiator (C) comprises a chemical polymerization initiator (C-2).

The amines can be classified into aliphatic amines and aromatic amines.

Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolaminedimethacrylate, N-ethyldiethanolaminedimethacrylate, triethanolaminemonomethacrylate, triethanolaminedimethacrylate, triethanolaminetrimethacrylate, triethanolamine, trimethylamine, triethylamine, and tributylamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate.

In view of imparting superior curability to the curable composition for dental use, preferred among these is at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Examples of sulfinic acid and salts thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate.

Examples of sulfites and bisulfites include sodium sulfite, potassium sulfite, calcium sulfite, ammonium sulfite, sodium bisulfite, and potassium bisulfite.

Examples of aldehydes include terephthalaldehyde and benzaldehyde derivatives. Examples of benzaldehyde derivatives include dimethylaminobenzaldehyde, p-methoxybenzaldehyde, p-ethoxybenzaldehyde, and p-n-octyloxybenzaldehyde.

Examples of thiourea compounds include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 3,3-dimethylethylenethiourea, and 4,4-dimethylethylenethiourea. In view of the curability of the curable composition for dental use, preferred among these is at least one selected from the group consisting of 1-(2-pyridyl)-2-thiourea, and 4,4-dimethylethylenethiourea.

Examples of organic phosphorus compounds include triphenylphosphine, 2-methyltriphenylphosphine, 4-methyltriphenylphosphine, 2-methoxytriphenylphosphine, 4-methoxytriphenylphosphine, tri-n-butylphosphine, triisobutylphosphine, and tri-t-butylphosphine.

Preferred as borate compounds are arylborate compounds.

Specific examples of arylborate compounds preferred for use are as follows. Examples of borate compounds with a single aryl group within a single molecule include trialkylphenylboron, trialkyl(p-chlorophenyl)boron, trialkyl(p-fluorophenyl)boron, trialkyl[3,5-bis(trifluoromethyl)phenyl]boron, trialkyl[3,5-bis(1,1,1,3,3,3-hexafluoro-2-methoxy-2-propyl)phenyl]boron, trialkyl(p-nitrophenyl)boron, trialkyl(m-nitrophenyl)boron, trialkyl(p-butylphenyl)boron, trialkyl(m-butylphenyl)boron, trialkyl(p-butyloxyphenyl)boron, trialkyl(m-butyloxyphenyl)boron, trialkyl(p-octyloxyphenyl)boron, and trialkyl(m-octyloxyphenyl)boron (the alkyl group is at least one selected from the group consisting of an n-butyl group, an n-octyl group, an n-dodecyl group, and similar groups), as well as salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of borate compounds with two aryl groups within a single molecule include dialkyldiphenylboron, dialkyldi(p-chlorophenyl)boron, dialkyldi(p-fluorophenyl)boron, dialkyldi[3,5-bis(trifluoromethyl)phenyl]boron, dialkyldi[3,5-bis(1,1,1,3,3,3-hexafluoro-2-methoxy-2-propyl)phenyl]boron, dialkyldi(p-nitrophenyl)boron, dialkyldi(m-nitrophenyl)boron, dialkyldi(p-butylphenyl)boron, dialkyldi(m-butylphenyl)boron, dialkyldi(p-butyloxyphenyl)boron, dialkyldi(m-butyloxyphenyl)boron, dialkyldi(p-octyloxyphenyl)boron, and dialkyldi(m-octyloxyphenyl)boron (the alkyl group is at least one selected from the group consisting of an n-butyl group, an n-octyl group, an n-dodecyl group, and similar groups), as well as salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of borate compounds with three aryl groups within a single molecule include monoalkyltriphenylboron, monoalkyltri(p-chlorophenyl)boron, monoalkyltri(p-fluorophenyl)boron, monoalkyltri[3,5-bis(trifluoromethyl)phenyl]boron, monoalkyltri[3,5-bis(1,1,1,3,3,3-hexafluoro-2-methoxy-2-propyl)phenyl]boron, monoalkyltri(p-nitrophenyl)boron, monoalkyltri(m-nitrophenyl)boron, monoalkyltri(p-butylphenyl)boron, monoalkyltri(m-butylphenyl)boron, monoalkyltri(p-butyloxyphenyl)boron, monoalkyltri(m-butyloxyphenyl)boron, monoalkyltri(p-octyloxyphenyl)boron, and monoalkyltri(m-octyloxyphenyl)boron (the alkyl group is one selected from an n-butyl group, an n-octyl group, an n-dodecyl group, and similar groups), as well as salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of borate compounds with four aryl groups within a single molecule include tetraphenylboron, tetrakis(p-chlorophenyl)boron, tetrakis(p-fluorophenyl)boron, tetrakis[3,5-bis(trifluoromethyl)phenyl]boron, tetrakis[3,5-bis(1,1,1,3,3,3-hexafluoro-2-methoxy-2-propyl)phenyl]boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, [3,5-bis(trifluoromethyl)phenyl]triphenylboron, (p-nitrophenyl)triphenylboron, (m-butyloxyphenyl)triphenylboron, (p-butyloxyphenyl)triphenylboron, (m-octyloxyphenyl)triphenylboron, and (p-octyloxyphenyl)triphenylboron, as well as salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

In view of storage stability, preferred among these arylborate compounds are borate compounds having three or four aryl groups within a single molecule. The arylborate compounds may be used alone, or two or more thereof may be used in combination.

Examples of barbituric acid and its derivatives include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, and thiobarbituric acid, as well as salts of these (particularly preferred are alkali metals or alkali earth metals). Examples of salts of barbituric acid include sodium 5-butylbarbiturate, sodium 1,3,5-trimethylbarbiturate, and sodium 1-cyclohexyl-5-ethylbarbiturate.

Examples of triazine compounds include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-($\alpha,\alpha,\beta$-trichloroethyl)-4,6-bis(trichloromethyl)-s-triazine, 2-styryl-4,6-bis(trichloromethyl)-s-triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimethoxyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N,N-bis(2-hydroxyethyl)amino}ethoxy]-4,6-bis(trichloromethyl)-s-triazine, 2-[2-{N-hydroxyethyl-N-ethylamino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N,N-diallylamino}ethoxy]-4,6-bis(trichloromethyl)-s-triazine.

The vanadium compounds are preferably vanadium compounds with valences of IV and/or V. Examples of vanadium compounds with valences of IV and/or V include vanadium(IV) oxide, vanadyl(IV) acetylacetonate, vanadyl oxalate, vanadyl sulfate, vanadium(IV) oxobis(1-phenyl-1,3-butanedionate), bis(maltolato)oxovanadium(IV), vanadium(V) oxide, sodium metavanadate, and ammonium metavanadate. In view of the curability of the curable composition for dental use, preferred for use is vanadyl(IV) acetylacetonate.

Examples of copper compounds include copper acetylacetonate, copper(II) acetate, copper oleate, copper(II) chloride, and copper(II) bromide. In view of the curability of the curable composition for dental use, preferred for use is at least one selected from the group consisting of copper acetylacetonate, and copper(II) acetate.

Examples of tin compounds include di-n-butyltin dimaleate, di-n-octyltin dimaleate, di-n-octyltin dilaurate, and di-n-butyltin dilaurate. Preferred for use is at least one selected from the group consisting of di-n-octyltin dilaurate, and di-n-butyltin dilaurate.

Examples of cobalt compounds include acetylacetone cobalt, cobalt acetate, cobalt oleate, cobalt chloride, and cobalt bromide.

Preferred examples of halogen compounds include dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Examples of thiol compounds include 3-mercaptopropyltrimethoxysilane, 2-mercaptobenzoxazole, decanethiol, and thiobenzoic acid.

More preferred among these polymerization accelerators (D) is at least one selected from the group consisting of an amine, a thiourea compound, a vanadium compound, and a copper compound. Among these, even more preferred for use as polymerization accelerator (D) is at least one selected from the group consisting of 1-(2-pyridyl)-2-thiourea, N,N-di(2-hydroxyethyl)-p-toluidine, ethyl 4-(N,N-dimethylamino)benzoate, 4,4-dimethylethylenethiourea, vanadyl(IV) acetylacetonate, copper acetylacetonate, and copper(II) acetate.

The content of the polymerization accelerator (D) in the curable composition for dental use is not particularly limited. However, in view of considerations such as the curability of the curable composition for dental use, the content of polymerization accelerator (D) is preferably 0.001 to 30 parts by mass relative to total 100 parts by mass of the polymerizable monomer (B). When the content of polymerization accelerator (D) is 0.001 parts or more by mass, polymerization can sufficiently proceed, preventing stickiness. In view of these, the content of polymerization accelerator (D) is more preferably 0.05 parts or more by mass, even more preferably 0.1 parts or more by mass, particularly preferably 0.3 parts or more by mass relative to total 100 parts by mass of the polymerizable monomer (B). When the content of polymerization accelerator (D) is 30 parts or less by mass, it is possible to prevent precipitation of polymerization accelerator (D) from the curable composition for dental use. In view of these, the content of polymerization accelerator (D) is more preferably 20 parts or less by mass, even more preferably 10 parts or less by mass, particularly preferably 5.0 parts or less by mass relative to total 100 parts by mass of the polymerizable monomer (B).

Preferably, the curable composition for dental use is essentially free of polyacids.

Examples of polyacids include polycarboxylic acid, polyphosphoric acid, polyphosphonic acid, polysulfonic acid, polyboric acid, polyacrylic acid, polymaleic acid, and polyvinylphosphonic acid.

As used herein, "essentially free of polyacids" means that the polyacid content in total 100 mass% of the curable composition for dental use is less than 5 mass%, preferably less than 1 mass%, more preferably less than 0.1 mass%, even more preferably less than 0.01 mass%, particularly preferably 0 mass%.

Preferably, the curable composition for dental use is provided as a kit that is used in a two-pack form comprising a first agent and a second agent in separate packages. In this case, it is more preferable that the curable composition for dental use be provided as a kit that is used as a two-paste kit with a first agent and a second agent provided in paste form.

When the curable composition for dental use is provided as a two-paste kit, each paste is kept isolated from the other during storage, and the two pastes are kneaded immediately before use to initiate chemical polymerization.

When the curable composition for dental use additionally comprises a photopolymerization initiator (C-1), it is preferable to cure the curable composition for dental use by promoting photopolymerization alongside chemical polymerization.

In the present invention, the chemical polymerization initiator (C-2) may be used with the polymerization accelerator (D) to provide a redox polymerization initiator. Here, in view of storage stability, the chemical polymerization initiator (C-2) and polymerization accelerator (D) should be stored in separate containers. In this case, the curable composition for dental use is provided preferably as a curable composition for dental use in which at least a first agent containing chemical polymerization initiator (C-2), and a second agent containing polymerization accelerator (D) are provided in separate packages.

<Filler (F)>

In view of considerations such as adjusting the paste properties of the curable composition for dental use before curing, and imparting radiopacity to the cured product or further increasing the strength of the cured product, the curable composition for dental use preferably comprises a filler (F), aside from glass (A).

Examples of fillers that may be used as filler (F) include organic fillers, inorganic fillers, and organic-inorganic composite fillers. The filler (F) may be used alone, or two or more thereof may be used in combination.

Examples of materials of the organic fillers include polymethylmethacrylate, polyethylmethacrylate, methyl methacrylate-ethyl methacrylate copolymers, crosslinked polymethylmethacrylate, crosslinked polyethylmethacrylate, polyesters, polyamides, polycarbonates, polyphenylene ethers, polyoxymethylene, polyvinyl chloride, polystyrene, polyethylene, polypropylene, chloroprene rubber, nitrile rubber, ethylene-vinyl acetate copolymers, styrene-butadiene copolymers, acrylonitrile-styrene copolymers, and acrylonitrile-styrene-butadiene copolymers. These may be used alone, or two or more thereof may be used as a mixture. The shape of organic filler is not particularly limited, and the organic filler may be appropriately selected from fillers of different shapes, such as irregularly shaped fillers, and spherical fillers. The particle diameter of the filler can also be selected as appropriate.

Examples of materials of the inorganic fillers include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, and strontium boroaluminosilicate glass. These may be used alone, or two or more thereof may be used as a mixture. The shape of inorganic filler is not particularly limited, and the inorganic filler may be appropriately selected from fillers of different shapes, such as irregularly shaped fillers, and spherical fillers. The particle diameter of the filler can also be selected as appropriate.

To adjust compatibility with the component (A) and component (B), the inorganic filler may be used after an optional surface treatment with a known surface treatment agent such as a silane coupling agent. Examples of such surface treatment agents include vinyltrimethoxysilane, vinyltriethoxysilane, vinyltrichlorosilane, vinyltri($\beta$-methoxyethoxy)silane, 3-methacryloyloxypropyltrimethoxysilane, 11-methacryloyloxyundecyltrimethoxysilane, 3-glycidoxypropyltrimethoxysilane, 3-mercaptopropyltrimethoxysilane, and 3-aminopropyltriethoxysilane.

The surface treatment can be carried out using any known method, including, for example, a method that adds the surface treatment agent by spraying it while vigorously stirring the inorganic filler, a method that disperses or dissolves the inorganic filler and the surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removing water. In all of these methods, applying heat in a temperature range of typically 50 to 150°C allows the reaction between the surface of inorganic filler and the surface treatment agent to proceed to completion and enable surface treatment.

The organic-inorganic composite fillers are fillers obtained by adding a monomer compound (for example, such as polymerizable monomer (B)) to the inorganic filler, polymerizing the mixture in paste form, and pulverizing the resulting material. The organic-inorganic composite filler may be, for example, a TMPT filler (a filler obtained by mixing trimethylolpropane trimethacrylate and a silica filler, and pulverizing the mixture after polymerization). The shape of organic-inorganic composite filler is not particularly limited, and the organic-inorganic composite filler may be appropriately selected from fillers of different shapes, such as irregularly shaped fillers, and spherical fillers. The particle diameter of the filler can also be selected as appropriate.

In view of considerations such as the handling properties of the curable composition for dental use and the mechanical strength and transparency of the cured product, the filler (F) has an average primary particle diameter of preferably 0.001 to 10 $\mu$m, more preferably 0.005 to 5.0 $\mu$m, even more preferably 0.01 to 4.0 $\mu$m, particularly preferably 0.04 to 3.0 $\mu$m. In this specification, the average primary particle diameter of filler (F) can be determined using a technique such as light microscopy or electron microscopy. Specifically, light microscopy is more convenient for particles with a particle size of 100 nm or larger, whereas electron microscopy is a more convenient method of particle diameter measurement for particles smaller than 100 nm.

The content of filler (F) is not particularly limited. However, in view of the handling properties of the curable composition for dental use and the strength of the cured product, the total amount of glass (A) and filler (F) is preferably 30 parts or more by mass, more preferably 40 parts or more by mass, even more preferably 50 parts or more by mass, and is preferably 1,000 parts or less by mass, more preferably 900 parts or less by mass, even more preferably 850 parts or less by mass relative to total 100 parts by mass of the polymerizable monomer (B).

When contained, the filler (F) is preferably 0.001 to 10 parts by mass, more preferably 0.005 to 5 parts by mass, even more preferably 0.1 to 3 parts by mass relative to 100 parts by mass of glass (A).

<Other Additives>

Optionally, the curable composition for dental use may additionally incorporate known additives, provided that the present invention can exhibit its effects. Examples of such additives include a polymerization inhibitor (E), antioxidants, pigments, dyes, ultraviolet absorbers, organic solvents, and thickeners, excluding the components described above. In this specification, "other additives" exclude polymerizable monomers.

Examples of polymerization inhibitor (E) include hydroquinone, hydroquinonemonomethyl ether, dibutylhydroquinone, dibutylhydroquinonemonomethyl ether, t-butylcatechol, 2-t-butyl-4,6-dimethylphenol, 2,6-di-t-butylphenol, and 3,5-di-t-butyl-4-hydroxytoluene. When the curable composition for dental use comprises a polymerization inhibitor (E), the content of the polymerization inhibitor (E) is preferably 0.001 to 1.0 parts by mass, more preferably 0.005 to 0.5 parts by mass, even more preferably 0.01 to 0.1 parts by mass relative to total 100 parts by mass of the polymerizable monomer (B).

In the curable composition for dental use, the total content of other additives is not particularly limited, provided that the present invention can exhibit its effects. However, in view of allowing the present invention to more readily exhibit its effects, the total content of other additives is preferably 20 mass% or less, more preferably 15 mass% or less, even more preferably 10 mass% or less, particularly preferably 5 mass% or less, most preferably 2 mass% or less, or 0 mass%, in total 100 mass% of the curable composition for dental use.

The curable composition for dental use is not particularly limited, as long as it comprises a glass (A), a polymerizable monomer (B), a polymerization initiator (C), and, optionally, a polymerization accelerator (D). However, in view of more effectively preventing a decrease in the strength and water resistance of the cured product, as well as opacification, the curable composition for dental use is preferably essentially free of polycarboxylic acids.

As used herein, "essentially free of polycarboxylic acids" has the same meaning as "essentially free of polyacids" described above, with the difference that it relates to the content of polycarboxylic acids.

In view of producing a cured product with more favorable strength and color tone, the curable composition for dental use is preferably essentially free of water.

As used herein, "essentially free of water" means that the content of water is 5.0 mass% or less, preferably 1.0 mass% or less, more preferably 0.5 mass% or less, even more preferably 0.1 mass% or less, particularly preferably 0.05 mass% or less in total 100 mass% of the curable composition for dental use.

The curable composition for dental use exhibits superior ion-releasing properties and mechanical strength in its cured product. This makes the curable composition for dental use suitable as a dental composite resin in applications where such advantages can be exploited, specifically, with optimum suitability for dental restorative filling materials, dental cements, and dental core build-up materials.

The curable composition for dental use is not particularly limited with respect to methods such as the way the respective components are mixed, except where the curable composition for dental use is used in a two-pack form comprising the first agent and second agent as described above. Accordingly, the curable composition for dental use may be produced using methods known to a person skilled in the art.

EXAMPLES

The following describes the present invention in greater detail by way of Examples. It should be noted, however, that the present invention is in no way limited by the following Examples.

The components used in Examples and Comparative Examples are described below, along with the abbreviations.

[Production Example A]

[Glass (A)]

For each component, the corresponding raw material, such as an oxide, fluoride, carbonate, nitrate, or hydroxide, was prepared. These were weighed to yield the composition shown in column A of Table 1 after vitrification. Following thorough mixing, the mixture was placed in a platinum crucible and melted in an electric furnace. During melting, the temperature was appropriately adjusted within a range of 1,000 to 1,500°C to ensure sufficient degassing. The melt was then stirred as needed to promote uniformity, followed by clarification and quenching to obtain the glass.

However, there were instances where glass was not obtained.

In Table 1, the row labeled "Vitrification" is marked "G" when glass was obtained, and "NG" when it was not obtained.

[Production Examples B to P]

The glass was obtained following the same technique as in Production Example A, except that the ratios of components were modified as shown in Table 1, columns B to P.

It should be noted, however, that, in Production Examples H, J, N, and P, crystal precipitation occurred, resulting in opaque glass that could not be used as a curable composition for dental use.

[Hydrophilic (Meth)Acrylic Monomer (B-1) Containing Two or More (Meth)Acryloyloxy Groups Within Single Molecule]

BMHPE: 1,2-bis(3-methacryloyloxy-2-hydroxypropoxy)ethane (manufactured by Shin-Nakamura Chemical Co., Ltd.)
GDM: glycerol dimethacrylate (manufactured by Shin-Nakamura Chemical Co., Ltd.)

[Hydrophobic (Meth)Acrylic Monomer (B-2) Containing Two or More (Meth)Acryloyloxy Groups within Single Molecule]

UDMA: 2,2,4-trimethylhexamethylenebis(2-carbamoyloxyethyl)dimethacrylate (manufactured by Kyoeisha Chemical Co., Ltd.)
Bis-GMA: 2,2-bis[4-(2-hydroxy-3-methacryloyloxypropoxy)phenyl]propane

[Mono(Meth)Acrylic Monomer (B-3) That Does Not Contain Any of Hydroxyl Group, Acidic Group, Amino Group, or Amide Group Within Single Molecule]

POBMA: m-phenoxybenzyl methacrylate (manufactured by Kyoeisha Chemical Co., Ltd.)
LMA: lauryl methacrylate (manufactured by Kyoeisha Chemical Co., Ltd.)

[Polymerization Initiator (C)]

<Photopolymerization Initiator (C-1)>

CQ: Camphorquinone
BAPO: bis(2,4,6-trimethylbenzoyl)phenylphosphine oxide

<Chemical Polymerization Initiator (C-2)>

THP: 1,1,3,3-tetramethylbutyl hydroperoxide

[Polymerization Accelerator (D)]

PDE: 4-N,N-dimethylaminoethyl benzoate
VOAA: vanadyl acetylacetonate
2-PyTU: 1-(2-pyridyl)-2-thiourea

[Polymerization Inhibitor (E)]

BHT: 3,5-di-t-butyl-4-hydroxytoluene

[Filler (F)]

Ar380: hydrophilic fumed silica AEROSIL® 380 (manufactured by Nippon Aerosil Co., Ltd., average primary particle diameter: 0.007 $\mu$m)

<Measurement of Average Particle Diameter of Glass (A)>

The glass successfully produced in Table 1 was pulverized with a ball mill to obtain pulverized glass. The average particle diameter of the pulverized glass was then measured by volume for 200 particles using a laser diffraction particle size analyzer (manufactured by Shimadzu Corporation, Model SALD-2300), with a 0.2% sodium hexametaphosphate aqueous solution used as dispersion medium. The results are presented in Table 1.

<Measurement of Refractive Index of Glass (A)>

The pulverized glass obtained in the manner described above was used for the measurement of refractive index, in compliance with the refractive index measurement method (Part 2: V-block method) of JIS B 7071-2:2018. The results are presented in Table 1.
For glass (A), a refractive index between 1.500 and 1.600 was considered acceptable from an aesthetic viewpoint.
The glass obtained in Production Example O had a refractive index exceeding 1.600, failing to provide transparency suitable for use as a curable composition for dental use.

<Eluted Aluminum Ion Amount ($W_{Al}$), Eluted Silicon Ion Amount ($W_{Si}$), and Eluted Strontium Ion Amount ($W_{Sr}$)>

Using 100 parts by mass of the pulverized glass obtained in the manner described above, a layer of silanol compound was formed on the surface of glass (A), and a silane-treated glass powder was obtained through intermolecular dehydrocondensation of the silanol groups. Specifically, a silane-treated glass powder was obtained by carrying out surface treatment with 1.5 parts by mass of $\gamma$-methacryloyloxypropyltrimethoxysilane, using an ordinary method.
This silane-treated glass powder was then measured or evaluated with respect to the amount of eluted ions and the ratio of eluted ion amounts, as follows.
The content of each ionic species in the sample was measured by inductively coupled plasma atomic emission spectrometry (ICP-AES), using the method described below.
A resin container was charged with 250 mg of the silane-treated glass powder and 4 mL of distilled water, and these were stirred at 300 rpm for 24 hours in a 37°C constant temperature bath. The resulting solution was then centrifuged (10,000 rpm, 5 min) using a tabletop high-speed microcentrifuge Himac CT15E manufactured by Hitachi Koki Co., Ltd. (now Koki Holdings Co., Ltd.), and the supernatant was filtered through a 0.22 $\mu$m PTFE (polytetrafluoroethylene) membrane filter to prepare a solution for measurement. To this solution, nitric acid and hydrofluoric acid were added, and the volume was adjusted with ultrapure water to prepare a test solution.
Subsequently, the test solution was measured with an ICP emission spectrometer (Agilent 5110, Model VDV, manufactured by Agilent Technologies, Inc. under this trade name) under the following conditions to determine the concentration of each ionic species in the test solution.

[Measurement Conditions]

Measurement mode: Axial
Read time (s): 20
Number of repeats: 3
Stabilization time (s): 15
High-speed pump (rpm): not set
Pump speed (rpm): 12
Output (kW): 1.2
Plasma gas flow (L/min): 12
Auxiliary gas flow rate (L/min): 1.0
Nebulizer gas flow rate (L/min): 0.7

<Ratio of Eluted Ion Amounts $(W_{Sr})/(W_{Al+Si})$>

The ratio of eluted ion amounts $(W_{Sr})/(W_{Al+Si})$ was calculated from the measured $W_{Al}$, $W_{Si}$, and $W_{Sr}$ ($\mu$g/g) using the following formulae (1) and (2). An eluted ion ratio of 10 or higher indicates a high demineralization inhibition rate.

$$W_{Al+Si} = W_{Al} + W_{Si} \qquad ... (1)$$

$$\text{Ratio of eluted ion amounts} = W_{Sr} / W_{Al+Si} \qquad ... (2)$$

[Table 1]

| Components | | | Production Examples | | | | | | | | | | | | | | | |
|---|---|---|---|---|---|---|---|---|---|---|---|---|---|---|---|---|---|---|
| | | | A | B | C | D | E | F | G | H | I | J | K | L | M | N | O | P |
| Raw materials of glass | Si | mass% | 11.2 | 10.0 | 12.0 | 10.2 | 7.0 | 11.1 | 12.6 | 9.4 | 11.0 | 9.4 | 12.5 | 4.7 | 10.2 | 10.2 | 10.2 | 12.6 |
| | Al | mass% | 6.0 | 11.2 | 2.4 | 10.9 | 4.8 | 9.2 | 2.5 | 4.8 | 5.8 | 3.2 | 7.2 | 4.8 | 10.9 | 11.1 | 11.1 | 4.0 |
| | Sr | mass% | 22.7 | 30.9 | 18.7 | 31.5 | 11.2 | 19.9 | 19.7 | 34.9 | 7.0 | 7.0 | 14.2 | 20.9 | 31.5 | 10.5 | 10.5 | 20.9 |
| | Zn | mass% | 7.0 | 3.9 | 13.9 | 3.9 | 20.1 | 3.0 | 11.0 | 1.1 | 12.2 | 31.6 | 1.1 | 3.2 | 3.9 | 13.2 | 13.2 | 3.2 |
| | B | mass% | 4.8 | 2.3 | 5.7 | 1.7 | 9.3 | 4.4 | 6.0 | 3.1 | 3.1 | 3.1 | 3.1 | 12.4 | 0.5 | 2.0 | 2.0 | 6.2 |
| | F | mass% | 23.7 | 15.1 | 21.2 | 15.4 | 13.2 | 29.8 | 20.4 | 27.1 | 27.1 | 28.2 | 28.5 | 21.1 | 15.4 | 44.1 | 7.9 | 21.9 |
| | Na | mass% | 0 | 0 | 0 | 0 | 0 | 0 | 0.3 | 0 | 0 | 0 | 0 | 0 | 0 | 0 | 0 | 3.0 |
| | O | mass% | 24.6 | 26.6 | 26.2 | 26.4 | 34.4 | 22.6 | 27.5 | 19.6 | 33.8 | 17.5 | 33.4 | 32.9 | 27.6 | 9.0 | 45.2 | 28.2 |
| Vitrification | | | G | G | G | G | G | G | G | NG | G | NG | G | G | G | NG | G | NG |
| Refractive index | | | 1.568 | 1.542 | 1.594 | 1.542 | 1.598 | 1.540 | 1.580 | - | 1.546 | - | 1.522 | 1.568 | 1.538 | - | > 1.6 | - |
| Average particle diameter | | $\mu$m | 2.2 | 2.2 | 1.8 | 2.2 | 2.2 | 2.4 | 2.2 | - | 2.2 | - | 2.4 | 2.2 | 2.2 | - | 2.1 | - |
| Amount of eluted ions $\mu$g/g | Si | | 40 | 40 | 260 | 26 | 30 | 150 | 320 | | 30 | | 170 | 40 | 26 | | 32 | |
| | Al | | 40 | 22 | 20 | 28 | 30 | 22 | 20 | | 30 | | 22 | 40 | 28 | | 22 | |
| | Sr | | 5200 | 4000 | 9600 | 5600 | 1100 | 4400 | 4000 | | 100 | | 4800 | 9600 | 450 | | 5100 | |
| Ratio of eluted ion amounts $(W_{Sr}/W_{Al+Si})$ | | | 65 | 65 | 34 | 104 | 18 | 26 | 12 | | 2 | | 25 | 120 | 8 | | 94 | |

*G: good; NG: not good

[Examples 1-1 to 1-7, 2-1 to 2-8, 3-1 to 3-2, and Comparative Examples 1-1 to 1-4]

The components shown in Tables 2, 3, and 4 were mixed at ordinary temperature (25°C) to prepare a paste, producing the curable composition for dental use of each Example and Comparative Example. The silane-treated glass powder prepared as described above was used for the glass.

The two materials in Examples 3-1 and 3-2 were kneaded using a kneading device composed of a polyolefin resin cartridge with a pair of identical cylinders arranged in parallel (total inner volume 5 mL, product code SDL 005-01-SI, manufactured by Sulzer Mixpac AG, Switzerland); a mixing unit attached to the front end of the cartridge; and a plunger unit with a pair of cylindrical plungers connected and fixed together and each plunger being fitted into a respective cartridge from the rear end of the cartridge (product code PED 005-01-SI, manufactured by Sulzer Mixpac AG, Switzerland). The pastes inside the cylinders were collected in equal volumes by pushing the plunger unit.

The mixing unit is a mixing tip with eight mixing vanes (element) (product code ML 2.5-08-S, manufactured by Sulzer Mixpac AG, Switzerland).

The resulting curable composition for dental use was measured or evaluated for demineralization inhibition rate, radiopacity, and flexural strength, as follows.

<Demineralization Inhibition Rate>

The labial surface of bovine mandibular incisors was polished under running water using #80 silicon carbide paper (manufactured by Nihon Kenshi Co., Ltd.) to expose a flat dentin surface. The exposed flat surface was further polished under running water with #1000 silicon carbide paper (manufactured by Nihon Kenshi Co., Ltd.) to prepare a polished surface. Following polishing, the bovine mandibular incisor was cut perpendicular to the polished surface using a diamond cutter, yielding one half as a control and the other as a test specimen.

For the control, the area except the 2 mm × 6 mm polished surface was masked with a nail coating. For the test specimen, a cavity measuring 2 mm × 6 mm at the base and 2 mm in depth was created adjacent the 2 mm × 6 mm polished surface, and this cavity was filled with the curable composition for dental use prepared in each Example and Comparative Example.

Once filled, the one-pack type curable compositions for dental use in Tables 2 and 3 were cured using a dental LED photoirradiator (PenCure 2000 manufactured by J. Morita Corp. under this trade name).

For the two-pack type curable compositions for dental use in Table 4, a mixture obtained by mixing the first and second agents was filled into the cavity, and the resulting test specimen was immersed in a 37°C constant temperature water bath to promote and achieve curing.

After curing, each test specimen was polished again under running water using #1000 silicon carbide paper (manufactured by Nihon Kenshi Co., Ltd.) to level the 2 mm × 6 mm polished surface with the cured product filling the cavity measuring 2 mm × 6 mm at the base.

To improve measurement accuracy by ensuring that any excess ion release from regions outside the measurement area is excluded from measurement, the area except the 2 mm × 6 mm polished surface and the surface of the cured product of the curable composition for dental use was masked with a nail coating.

Using these test specimens, the amount of ion release was measured, and the demineralization inhibition rate was calculated, as follows.

The control and test specimen were placed separately in a polystyrene 12-well plate (wells measuring 25 mm in diameter × 17 mm) with the polished surfaces facing upward. To each well, 4 ml of divalent cation-free phosphate buffer PBS (-) was added, and the plate was maintained in a 37°C thermostatic chamber for 24 hours. Following this period, the control and test specimen were removed, and rinsed with ion-exchanged water. After removing moisture, these were placed separately in another polystyrene 12-well plate (wells measuring 25 mm in diameter × 17 mm), and 4 ml of 0.5 mol/L acetate buffer (pH = 4.6) was added to each well. The plate was then maintained in a 37°C thermostatic chamber for 24 hours.

After this period, the 0.5 mol/L acetate buffer was collected from each well, and 30 $\mu$l of a 0.1 mol/L potassium chloride aqueous solution, serving as an ionic strength stabilizer, was added to 0.3 ml of this acetate buffer. The resulting solution was shaken to ensure uniformity, yielding a demineralization solution. The concentration of eluted calcium ions in the demineralization solution was then measured using a calcium ion meter (LAQUA twin manufactured by Horiba Ltd. under this trade name). The concentration of eluted calcium ions was designated as $M_0$ for the control, and $M_1$ for the test specimen.

Subsequently, calcium ion solutions were prepared by adding 30 $\mu$l of a 0.1 mol/L potassium chloride aqueous solution to each 300 ml of solutions that had been diluted with 0.5 mol/L acetate buffer (pH = 4.6) to yield calcium ion concentrations of 2.5, 5.0, 10.0, 20.0, and 40.0 mmol/L. The concentrations of calcium ions in these calcium ion solutions were then measured using a calcium ion meter (LAQUA twin manufactured by Horiba Ltd. under this trade name), and a calibration curve, passing through the origin, was created by plotting the adjusted calcium ion concentrations (2.5, 5.0, 10.0, 20.0, 40.0 mmol/L) on the horizontal axis and the measured calcium ion concentrations on the vertical axis. The slope of this calibration curve is indicated by "A" in the formulae (3) and (4) below. These formulae (3) and (4) were used to determine the amounts of eluted calcium ions $Ca_0$ and $Ca_1$ ($\mu$mol/cm$^2$).

Here, the area of the 2 mm × 6 mm polished surface actually measured is indicated by $S_0$ for the control and $S_1$ for the test specimen.

$$\text{Amount of eluted Ca ions (control) } Ca_0 = (M_0/A) \times 4 \text{ (ml) } / S_0 \quad \text{... (3)}$$

$$\text{Amount of eluted Ca ions (test specimen) } Ca_1 = (M_1/A) \times 4 \text{ (ml) } / S_1 \quad \text{... (4)}$$

The demineralization inhibition rate was determined from the calculated values of $Ca_0$ and $Ca_1$, using the following formula (5).

$$\text{Demineralization inhibition rate (\%) } = (Ca_0 - Ca_1) / Ca_0 \quad \text{... (5)}$$

<Radiopacity >

The curable compositions for dental use prepared in Examples and Comparative Examples were prepared into cured specimens (1.5 mm in diameter × 1.0 mm) according to the type of formulation, as follows.

The one-pack type curable compositions for dental use in Tables 2 and 3 were cured into test specimens using a dental LED photoirradiator (PenCure 2000 manufactured by J. Morita Corp. under this trade name).

For the two-pack type curable compositions for dental use in Table 4, a mixture obtained by mixing the first and second agents was immersed in a 37°C constant temperature water bath to promote curing and prepare cured specimens.

Each test specimen was placed next to an aluminum step wedge (control) at the center of an X-ray film (Ultra-Speed Dental Film (occlusal) DF-50 manufactured by Carestream Health Co., Ltd. under this trade name), and irradiated with X-rays using a digital X-ray imager (Max DC-70 manufactured by J. Morita Corp. under this trade name) with a target-to-film distance of 400 mm and a tube voltage of 70 kV.

After irradiation, the film was developed, fixed, and dried. The image density of the test specimen was then measured at 20 points using an optical densitometer (DENSITOMETER PDA-85 manufactured by Konica, currently Konica Minolta Inc.; a measurement area of Ø = 3 mm). By comparing the image density of the test specimen with that of the aluminum step wedge at each thickness, the radiopacity of the test specimen was determined in terms of an equivalent thickness of the aluminum step wedge (n = 1).

The preferred radiopacity is 1.0 mm or more, more preferably 1.3 mm or more, even more preferably 1.5 mm or more. The radiopacity may be 2.0 mm or more, or 3.0 mm or more.

The radiopacity is indicated as "≥ 1" when it is 1 mm or greater, and "< 1" when it is below 1.

\<Flexural Strength\>

The flexural strength was evaluated by carrying out a flexure test (three-point flexural test) in compliance with ISO 4049:2019.

The kneaded product of each curable composition for dental use prepared in Examples and Comparative Examples was filled into a SUS mold (2 mm wide × 2 mm thick × 25 mm long) and compressed vertically from both sides with glass slides.

For the one-pack type curable compositions for dental use in Tables 2 and 3, the curable composition for dental use was cured by 10 seconds of light irradiation using a dental LED photoirradiator (PenCure 2000 manufactured by J. Morita Corp. under this trade name). The resulting cured product was then removed from the mold and immersed in a 37°C constant temperature water bath.

For the two-pack type curable compositions for dental use in Table 4, a mixture obtained by mixing the first and second agents was filled into the SUS mold and compressed vertically from both sides with glass slides. The resulting product was removed from the mold and immersed in a 37°C constant temperature water bath to promote curing and obtain a cured product.

These curable compositions were immersed in the 37°C constant temperature water baths for one day (1 d) and one week (1 w). The resulting cured products were subjected to a three-point flexural test at a span length of 20 mm and a crosshead speed of 2 mm/min using a universal testing machine (Autograph® AG-I 100kN manufactured by Shimadzu Corporation under this trade name), and the flexural strength and flexural modulus were measured (arithmetic mean for n = 5).

For one day of immersion in the 37°C constant temperature water bath, the preferred flexural strength (1 d) is 80 MPa or more, more preferably 100 MPa or more, even more preferably 120 MPa.

For one week of immersion in the 37°C constant temperature water bath, the preferred flexural strength (1 w) is 50 MPa or more, more preferably 80 MPa or more, even more preferably 100 MPa.

[Table 2]

| Components (parts by mass) | | | Examples | | | | | | | Comparative Examples | | | |
|---|---|---|---|---|---|---|---|---|---|---|---|---|---|
| | | | 1-1 | 1-2 | 1-3 | 1-4 | 1-5 | 1-6 | 1-7 | 1-1 | 1-2 | 1-3 | 1-4 |
| A | Glass | Type | A | B | C | D | E | F | G | I | K | L | M |
| | | | 69.0 | 69.0 | 69.0 | 69.0 | 69.0 | 69.0 | 69.0 | 69.0 | 69.0 | 69.0 | 69.0 |
| B-1 | BMHPE | | 32.5 | 32.5 | 32.5 | 32.5 | 32.5 | 32.5 | 32.5 | 32.5 | 32.5 | 32.5 | 32.5 |
| | GDM | | 0.0 | 0.0 | 0.0 | 0.0 | 0.0 | 0.0 | 0.0 | 0.0 | 0.0 | 0.0 | 0.0 |
| B-2 | UDMA | | 51.5 | 51.5 | 51.5 | 51.5 | 51.5 | 51.5 | 51.5 | 51.5 | 51.5 | 51.5 | 51.5 |
| | Bis-GMA | | 0.0 | 0.0 | 0.0 | 0.0 | 0.0 | 0.0 | 0.0 | 0.0 | 0.0 | 0.0 | 0.0 |
| B-3 | POBMA | | 16.0 | 16.0 | 16.0 | 16.0 | 16.0 | 16.0 | 16.0 | 16.0 | 16.0 | 16.0 | 16.0 |
| | LMA | | 0.0 | 0.0 | 0.0 | 0.0 | 0.0 | 0.0 | 0.0 | 0.0 | 0.0 | 0.0 | 0.0 |
| C-1 | CQ | | 0.10 | 0.10 | 0.10 | 0.10 | 0.10 | 0.10 | 0.10 | 0.10 | 0.10 | 0.10 | 0.10 |
| | BAPO | | 0.00 | 0.00 | 0.00 | 0.00 | 0.00 | 0.00 | 0.00 | 0.00 | 0.00 | 0.00 | 0.00 |
| D | PDE | | 0.15 | 0.15 | 0.15 | 0.15 | 0.15 | 0.15 | 0.15 | 0.15 | 0.15 | 0.15 | 0.15 |
| E | BHT | | 0.05 | 0.05 | 0.05 | 0.05 | 0.05 | 0.05 | 0.05 | 0.05 | 0.05 | 0.05 | 0.05 |

(continued)

| Components (parts by mass) | | | Examples | | | | | | | Comparative Examples | | | |
|---|---|---|---|---|---|---|---|---|---|---|---|---|---|
| | | | 1-1 | 1-2 | 1-3 | 1-4 | 1-5 | 1-6 | 1-7 | 1-1 | 1-2 | 1-3 | 1-4 |
| F | Ar380 | | 3.5 | 3.5 | 3.5 | 3.5 | 3.5 | 3.5 | 3.5 | 3.5 | 3.5 | 3.5 | 3.5 |
| Demineralization inhibition rate | | % | 33 | 27 | 65 | 33 | 15 | 26 | 15 | 4 | 9 | 28 | 4 |
| Radiopacity | | mm | ≥ 1 | ≥ 1 | ≥ 1 | ≥ 1 | ≥ 1 | ≥ 1 | ≥ 1 | ≥ 1 | ≥ 1 | ≥ 1 | ≥ 1 |
| Flexural strength (MPa) | | 1 d | 102 | 102 | 103 | 92 | 80 | 80 | 100 | 89 | 92 | 50 | 92 |
| | | 1 w | 91 | 90 | 91 | 89 | 77 | 55 | 91 | 78 | 88 | 42 | 89 |

[Table 3]

| Components (parts by mass) | | | Examples | | | | | | | |
|---|---|---|---|---|---|---|---|---|---|---|
| | | | 2-1 | 2-2 | 2-3 | 2-4 | 2-5 | 2-6 | 2-7 | 2-8 |
| A | Glass | Type | A | A | A | A | A | A | A | A |
| | | | 365.0 | 900.0 | 69.0 | 69.0 | 69.0 | 69.0 | 69.0 | 69.0 |
| B-1 | BMHPE | | 32.5 | 32.5 | 15.0 | 50.0 | 32.5 | 0.0 | 38.7 | 0.0 |
| | GDM | | 0.0 | 0.0 | 0.0 | 0.0 | 0.0 | 32.5 | 0.0 | 0.0 |
| B-2 | UDMA | | 51.5 | 51.5 | 65.0 | 38.0 | 25.0 | 0.0 | 61.3 | 76.3 |
| | Bis-GMA | | 0.0 | 0.0 | 0.0 | 0.0 | 0.0 | 51.5 | 0.0 | 0.0 |
| B-3 | POBMA | | 16.0 | 16.0 | 20.0 | 12.0 | 42.5 | 0.0 | 0.0 | 23.7 |
| | LMA | | 0.0 | 0.0 | 0.0 | 0.0 | 0.0 | 16.0 | 0.0 | 0.0 |
| C-1 | CQ | | 0.10 | 0.10 | 0.10 | 0.10 | 0.10 | 0.10 | 0.10 | 0.10 |
| | BAPO | | 0.00 | 0.50 | 0.00 | 0.00 | 0.00 | 0.00 | 0.00 | 0.00 |
| D | PDE | | 0.15 | 0.15 | 0.15 | 0.15 | 0.15 | 0.15 | 0.15 | 0.15 |
| E | BHT | | 0.00 | 0.05 | 0.05 | 0.05 | 0.05 | 0.05 | 0.05 | 0.05 |
| F | Ar380 | | 2.5 | 0.1 | 3.5 | 3.5 | 3.5 | 3.5 | 3.5 | 3.5 |
| Demineralization inhibition rate | | % | 65 | 70 | 28 | 42 | 32 | 22 | 21 | 14 |
| Radiopacity | | mm | ≥ 1 | ≥ 1 | ≥ 1 | ≥ 1 | ≥ 1 | ≥ 1 | ≥ 1 | ≥ 1 |
| Flexural strength (MPa) | | 1 d | 120 | 128 | 110 | 92 | 95 | 100 | 81 | 115 |
| | | 1 w | 108 | 115 | 105 | 81 | 89 | 89 | 70 | 111 |

[Table 4]

| Components (parts by mass) | | | Example 3-1 | | | Example 3-2 | | |
|---|---|---|---|---|---|---|---|---|
| | | | First agent | Second agent | Mixture | First agent | Second agent | Mixture |
| A | Glass | Type | A | A | A | A | A | A |
| | | | 69.0 | 69.0 | 69.0 | 69.0 | 69.0 | 69.0 |
| B-1 | BMHPE | | 32.5 | 32.5 | 32.5 | 32.5 | 32.5 | 32.5 |
| | GDM | | 0.0 | 0.0 | 0.0 | 0.0 | 0.0 | 0.0 |
| B-2 | UDMA | | 51.5 | 51.5 | 51.5 | 51.5 | 51.5 | 51.5 |
| | Bis-GMA | | 0.0 | 0.0 | 0.0 | 0.0 | 0.0 | 0.0 |

(continued)

| Components (parts by mass) | | | Example 3-1 | | | Example 3-2 | | |
|---|---|---|---|---|---|---|---|---|
| | | | First agent | Second agent | Mixture | First agent | Second agent | Mixture |
| B-3 | POBMA | | 16.0 | 16.0 | 16.0 | 16.0 | 16.0 | 16.0 |
| | LMA | | 0.0 | 0.0 | 0.0 | 0.0 | 0.0 | 0.0 |
| C-1 | CQ | | 0.00 | 0.10 | 0.050 | 0.00 | 0.00 | 0.000 |
| | BAPO | | 0.00 | 0.00 | 0.000 | 0.00 | 0.00 | 0.000 |
| C-2 | THP | | 3.00 | 0.00 | 1.500 | 3.00 | 0.00 | 1.500 |
| D | PDE | | 0.15 | 0.00 | 0.075 | 0.00 | 0.00 | 0.000 |
| | VOAA | | 0.00 | 0.0750 | 0.0375 | 0.00 | 0.0750 | 0.0375 |
| | 2-PyTU | | 0.00 | 1.50 | 0.750 | 0.00 | 1.50 | 0.750 |
| E | BHT | | 0.05 | 0.05 | 0.05 | 0.05 | 0.05 | 0.05 |
| F | Ar380 | | 3.5 | 3.5 | 3.5 | 3.5 | 3.5 | 3.5 |
| Demineralization inhibition rate | | % | 32 | | | 31 | | |
| Radiopacity | | mm | $\geq 1$ | | | $\geq 1$ | | |
| Flexural strength (MPa) | | 1 d | 105 | | | 101 | | |
| | | 1 w | 92 | | | 89 | | |

The results in Tables 2, 3, and 4 confirmed excellent demineralization inhibition rate, radiopacity, and flexural strength in the one-pack type and two-pack type curable compositions for dental use of Examples.

In contrast, Comparative Example 1-3, with a high boron content, showed low flexural strength.

Comparative Examples 1-1, 1-2, and 1-4, low in strontium, zinc, and boron, respectively, showed low demineralization inhibition rate.

INDUSTRIAL APPLICABILITY

The curable composition for dental use can be suitably used for dental composite resins, dental restorative filling materials, dental cements, and dental core build-up materials, with particular suitability for dental restorative filling materials, dental cements, and dental core build-up materials.

**Claims**

1. A curable composition for dental use, comprising a glass (A), a polymerizable monomer (B), and a polymerization initiator (C),

   wherein the glass (A) comprises strontium, zinc, boron, and fluorine, and
   the strontium content is 8 to 33 mass%, the zinc content is 2.0 to 25 mass%, the boron content is 1.0 to 11 mass%, the fluorine content is 9 to 35 mass%, and the sodium content is 0 to 0.5 mass%, expressed as mass percentages of the constituent elements in the total mass of the glass (A).

2. The curable composition for dental use according to claim 1, wherein the polymerizable monomer (B) comprises a hydrophilic (meth)acrylic monomer (B-1) containing two or more (meth)acryloyloxy groups within a single molecule, and a hydrophobic (meth)acrylic monomer (B-2) containing two or more (meth)acryloyloxy groups within a single molecule.

3. The curable composition for dental use according to claim 1 or 2, wherein the polymerizable monomer (B) further comprises a mono(meth)acrylic monomer (B-3) that does not contain any of a hydroxyl group, an acidic group, an amino group, or an amide group within a single molecule.

4. The curable composition for dental use according to claim 1 or 2, which comprises the following in total 100 parts by mass of the polymerizable monomer (B):

   5 to 55 parts by mass of a hydrophilic (meth)acrylic monomer (B-1) containing two or more (meth)acryloyloxy groups within a single molecule;
   25 to 70 parts by mass of a hydrophobic (meth)acrylic monomer (B-2) containing two or more (meth)acryloyloxy groups within a single molecule; and
   0 to 55 parts by mass of a mono(meth)acrylic monomer (B-3) that does not contain any of a hydroxyl group, an acidic group, an amino group, or an amide group within a single molecule.

5. The curable composition for dental use according to claim 1 or 2,

   wherein the glass (A) further comprises aluminum and silicon, and
   the ratio of the amount of eluted strontium ions ($W_{Sr}$) to the sum of the amounts of eluted aluminum ions and eluted silicon ions ($W_{Al+Si}$) is 10 or more (($W_{Sr}$)/($W_{Al+Si}$)) when a silane-treated glass powder obtained by surface treatment of 100 parts by mass of the glass (A) in pulverized form with 1.5 parts by mass of $\gamma$-methacryloyloxypropyltrimethoxysilane is immersed in 37°C distilled water for 24 hours at a concentration of 5 mass%.

6. The curable composition for dental use according to claim 1 or 2, which is essentially free of polyacids.

7. The curable composition for dental use according to claim 1 or 2, wherein the polymerization initiator (C) comprises a chemical polymerization initiator (C-2).

8. The curable composition for dental use according to claim 1 or 2, which further comprises a polymerization accelerator (D).

9. The curable composition for dental use according to claim 1 or 2, which comprises a first agent and a second agent in separate packages.

10. The curable composition for dental use according to claim 9, wherein the first agent comprises a chemical polymerization initiator (C-2), and the second agent comprises a polymerization accelerator (D).

## INTERNATIONAL SEARCH REPORT

| International application No. |
|---|
| **PCT/JP2024/028583** |

| A. | CLASSIFICATION OF SUBJECT MATTER |
|---|---|

***A61K 6/836***(2020.01)i; ***A61K 6/62***(2020.01)i; ***A61K 6/887***(2020.01)i
FI:   A61K6/836; A61K6/887; A61K6/62

According to International Patent Classification (IPC) or to both national classification and IPC

| B. | FIELDS SEARCHED |
|---|---|

Minimum documentation searched (classification system followed by classification symbols)

    A61K6/836; A61K6/62; A61K6/887

Documentation searched other than minimum documentation to the extent that such documents are included in the fields searched

    Published examined utility model applications of Japan 1922-1996
    Published unexamined utility model applications of Japan 1971-2024
    Registered utility model specifications of Japan 1996-2024
    Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2023/120611 A1 (KURARAY NORITAKE DENTAL INC.) 29 June 2023 (2023-06-29) entire text | 1-10 |
| A | WO 2022/209201 A1 (GC CORPORATION) 06 October 2022 (2022-10-06) entire text | 1-10 |
| A | JP 2022-29160 A (GC CORPORATION) 17 February 2022 (2022-02-17) entire text | 1-10 |
| A | JP 2000-143430 A (CARL-ZEISS-STIFTUNG) 23 May 2000 (2000-05-23) entire text | 1-10 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 September 2024** | **29 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/028583**

| Patent document cited in search report | Publication date (day/month/year) | Patent family member(s) | Publication date (day/month/year) |
|---|---|---|---|
| WO 2023/120611 A1 | 29 June 2023 | (Family: none) | |
| WO 2022/209201 A1 | 06 October 2022 | US 2024/0164994 A1 <br> entire text <br> EP 4316454 A1 | |
| JP 2022-29160 A | 17 February 2022 | US 2022/0040048 A1 <br> entire text <br> EP 3950615 A1 | |
| JP 2000-143430 A | 23 May 2000 | US 6297181 B1 <br> entire text <br> EP 997132 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000159621 A

**Non-patent literature cited in the description**

- **OSVALDO ZMENER et al.** Resistance against bacterial leakage of four luting agents used for cementation of complete cast crowns. *American Journal of Dentistry, (US)*, February 2014, vol. 27 (1), 51-55